# EUROPEAN PATENT SPECIFICATION

(11) **EP 3 090 044 B1**
(45) Date of publication and mention of the grant of the patent: **03.11.2021**
(21) Application number: 14875819.6
(22) Date of filing: 18.09.2014
(51) Int. Cl.: C12Q 1/68, C12N 15/00, C07H 21/04

(54) **MULTIPLEX GUIDE RNAS**
MULTIPLEX-FÜHRUNGS-RNAS
ARN GUIDES MULTIPLEX

(30) Priority: 26.12.2013 US 201361921007 P; 23.01.2014 US 201461930782 P; 14.03.2014 US 201414211117; 14.03.2014 WO PCT/US2014/029068; 14.03.2014 WO PCT/US2014/028630; 14.03.2014 WO PCT/US2014/029304; 23.04.2014 WO PCT/US2014/035162
(43) Date of publication of application: 09.11.2016
(60) Divisional application: 21191144.1
(73) Proprietor: The General Hospital Corporation, Boston, MA 02114 (US)
(72) Inventor: TSAI, Shengdar, Charlestown, Massachusetts 02129 (US); JOUNG, Keith J., Winchester, Massachussetts 01890 (US)
(74) Representative: Forresters IP LLP
(86) International application number: PCT/US2014/056416
(87) International publication number: WO 2015/099850

(56) References cited:
- WO-A1-2013/176772
- WO-A1-2015/153940
- WO-A2-2011/143124
- WO-A2-2014/152432
- MORGAN L MAEDER ET AL: "CRISPR RNA-guided activation of endogenous human genes", NATURE METHODS, vol. 10, no. 10, 25 July 2013 (2013-07-25) , pages 977-979, XP055291599, ISSN: 1548-7091, DOI: 10.1038/nmeth.2598
- P. MALI ET AL: "RNA-Guided Human Genome Engineering via Cas9", SCIENCE, vol. 339, no. 6121, 15 February 2013 (2013-02-15), pages 823-826, XP055337932, ISSN: 0036-8075, DOI: 10.1126/science.1232033
- HAURWITZ, R.: 'The CRISPR endoribonuclease Csy4 utilizes unusual sequenceand structure- specific mechanisms to recognize and process crRNAs' THESIS. 08 May 2012, UNIVERSITY OF CALIFORNIA, BERKELEY, pages 1 - 120, XP055293745 Retrieved from the Internet: <URL:http://escholarship.org/uc/item/0rh594 0p>
- NISSIM ET AL.: 'Multiplexed and Programmable Regulation of Gene Networks with an Integrated RNA and CRISPR/Cas Toolkit in Human Cells' MOLECULAR CELL vol. 54, 22 May 2014, pages 698 - 710, XP029028594
- None

## Description

### CROSS-REFERENCE TO RELATED APPLICATIONS

This application claims the benefit of Patent Applications 61/921,007, filed on December 26, 2013; 14/211,117, filed on March 14, 2014; PCT/US2014/029068, filed on March 14, 2014; PCT/US2014/028630, filed on March 14, 2014; PCT/US2014/035162, filed on March 23, 2014; PCT/US2014/029304, filed on March 14, 2014; and 61/930,782, filed on January 23, 2014.

### FEDERALLY SPONSORED RESEARCH OR DEVELOPMENT

This invention was made with Government support under Grant No. DP1 GM105378 awarded by the National Institutes of Health. The Government has certain rights in the invention.

### TECHNICAL FIELD

Described are methods and constructs for the multiplex expression of highly active CRISPR guide RNAs (gRNAs) from RNA Polymerase II and III promoters, optionally in mammalian cells.

### BACKGROUND

The Cas9 nuclease forms the basis of a programmable RNA-guided clustered, regularly interspaced, short palindromic repeats (CRISPR)/CRISPR-associated (Cas) system (Wiedenheft et al., Nature 482, 331-338 (2012); Horvath et al., Science 327, 167-170 (2010); Terns et al., Curr Opin Microbiol 14, 321-327 (2011)) that can be used to create site-specific breaks in target DNA sequences in vitro, in mammalian cells, and in living model organisms such as zebrafish (Wang et al., Cell 153, 910-918 (2013); Shen et al., Cell Res (2013); Dicarlo et al., Nucleic Acids Res (2013); Jiang et al., Nat Biotechnol 31, 233-239 (2013); Jinek et al., Elife 2, e00471 (2013); Hwang et al., Nat Biotechnol 31, 227-229 (2013); Cong et al., Science 339, 819-823 (2013); Mali et al., Science 339, 823-826 (2013c); Cho et al., Nat Biotechnol 31, 230-232 (2013); Gratz et al., Genetics 194(4):1029-35 (2013)). A short ∼100 nt guide RNA (gRNA) complexes with Cas9 and directs the nuclease to a specific target DNA site; targeting is mediated by a sequence of at least 17-20 nucleotides (nts) at the 5' end of the gRNA, which are designed to be complementary to and interact via simple base pair complementarity between the first 17-20 nucleotides of an engineered gRNA and the complementary strand of a target genomic DNA sequence of interest that lies next to a protospacer adjacent motif (PAM), e.g., a PAM matching the sequence NGG or NAG (Shen et al., Cell Res (2013); Dicarlo et al., Nucleic Acids Res (2013); Jiang et al., Nat Biotechnol 31, 233-239 (2013); Jinek et al., Elife 2, e00471 (2013); Hwang et al., Nat Biotechnol 31, 227-229 (2013); Cong et al., Science 339, 819-823 (2013); Mali et al., Science 339, 823-826 (2013c); Cho et al., Nat Biotechnol 31, 230-232 (2013); Jinek et al., Science 337, 816-821 (2012)). gRNAs can also direct catalytically inactivated Cas9 proteins (known as dCas9, see Jinek et al., Science 337:816-821 (2012)) that are in turn fused to effector domains (e.g., a transcriptional activation domain) see, e.g., USSN 61/799,647, filed on March 15, 2013, and 61/838,148, filed on June 21, 2013. These latter systems enable RNA-guided recruitment of heterologous effector domains to genomic loci of interest.

Maeder *et al* (2013) discloses an effect of multiple gRNAs binding to a single promoter. Mali *et al* (2013) discloses the simultaneous introduction of multiple gRNAs. WO2015153940 discloses expression of gRNAs using an RNA pol II promoter. WO2013176772 discloses DNA-targeting RNA comprising a first segment complementary to a target sequence and a second segment interacting with a site-directed modifying polypeptide. WO2011143124 discloses an enzymatically inactive variant of Csy4 endoribonuclease. Haurwitz, R (2012), WO2014152432 discloses fusion proteins comprising dCas9. Nissim *et al* (2014) is the scientific publication of WO2015153940.

### SUMMARY

The invention is defined in the claims. The present invention is based, at least in part, on the discovery that Csy4, an endoribonuclease that recognizes a short RNA hairpin sequence, can be used to cleave out multiple functional gRNAs encoded on a single longer RNA transcript (produced from an RNA pol II or III promoter) in which the individual gRNAs are separated by Csy4 cleavage sites.

Thus in a first aspect the disclosure provides deoxyribonucleic acid (DNA) molecules comprising a plurality of sequences encoding guide RNAs (gRNAs), wherein each gRNA is flanked by at least one Csy4 cleavage sequence comprising or consisting of the sequence
GTTCACTGCCGTATAGGCAG (SEQ ID NO:1) or
GTTCACTGCCGTATAGGCAGCTAAGAAA (SEQ ID NO:2).

In some aspects of the disclosure the DNA molecule is operably linked to a promoter sequence.

In some aspects of the disclosure the DNA molecule includes two, three, or more gRNA sequences, each flanked by at least one Csy4 cleavage sequence.

In some aspects of the disclosure, the promoter sequence is a RNA Polymerase II (Pol II) promoter or Pol III promoter, preferably a RNA Pol II promoter. In some embodiments the Pol II promoter is selected from the group consisting of CAG, EF1A, CAGGS, PGK, UbiC, CMV, B29, Desmin, Endoglin, FLT-1, GFPA, and SYN1 promoters.

In another aspect of the disclosure the invention provides a DNA molecule comprising a promoter sequence linked with one, two, three or more cassettes comprising: a sequence encoding a guide RNA, i.e., a sequence of about 100 nts, e.g., 95-300 nts, e.g., 95-105 nts for an *S. Pyogenes-based* system, linked to a Csy4 cleavage site, e.g., SEQ ID NO:1 or 2.

In some aspects of the disclosure the DNA molecule comprises a Pol II promoter, operably linked to a first sequence encoding a first guide RNA linked to a Csy4 cleavage site, linked to a second sequence encoding a second guide RNA linked to a Csy4 cleavage site, linked to a third sequence encoding a third guide RNA linked to a Csy4 cleavage site. In some embodiments, further guide RNAs linked to Csy4 cleavage sites are included. For example, the DNA molecule can have the following structure:
Promoter - C4 - gRNA - C4 - gRNA - C4 - gRNA - C4
Promoter - C4 - gRNA - C4 - gRNA - C4 - gRNA - C4 - gRNA - C4
Promoter - C4 - gRNA - C4 - gRNA - C4 - gRNA - C4 - gRNA - C4 - gRNA C4
And so on. In this illustration C4 is a sequence encoding a Csy4 RNA cleavage site and gRNA is a sequence encoding a guide RNA.

In some embodiments, the the Cas9 sgRNA comprises the sequence:
(X₁₇₋₂₀) GUUUUAGAGCUAUGCUGUUUUG(X_{N}) (SEQ ID NO:4);
(X₁₇₋₂₀)GUUUUAGAGCUA (SEQ ID NO:5);
(X₁₇₋₂₀) GUUUUAGAGCUAUGCUGUUUUG (SEQ ID NO:6);
(X₁₇₋₂₀)GUUUUAGAGCUAUGCU (SEQ ID NO:7);
(X₁₇₋₂₀)GUUUUAGAGCUAGAAAUAGCAAGUUAAAAUAAGGCUAGUCCG (X_{N}) (SEQ ID NO:8);

(X17-20)GUUUUAGAGCUAUGCUGAAAAGCAUAGCAAGUUAAAAUAAGGCUAGUC
CGUUAUC(XN) (SEQ ID NO:9);

(X17-20)GUUUUAGAGCUAUGCUGUUUUGGAAACAAAACAGCAUAGCAAGUUAAA
AUAAGGCUAGUCCGUUAUC(XN) (SEQ ID NO:10);

(X17-20)GUUUUAGAGCUAGAAAUAGCAAGUUAAAAUAAGGCUAGUCCGUUAUCA
ACUUGAAAAAGUGGCACCGAGUCGGUGC(XN) (SEQ ID NO:11),

(X17-20)GUUUAAGAGCUAGAAAUAGCAAGUUUAAAUAAGGCUAGUCCGUUAUCA
ACUUGAAAAAGUGGCACCGAGUCGGUGC(SEQ ID NO:12);

(X17-20)GUUUUAGAGCUAUGCUGGAAACAGCAUAGCAAGUUUAAAUAAGGCUAG
UCCGUUAUCAACUUGAAAAAGUGGCACCGAGUCGGUGC (SEQ ID NO:13); or

(X17-20)GUUUAAGAGCUAUGCUGGAAACAGCAUAGCAAGUUUAAAUAAGGCUAG
UCCGUUAUCAACUUGAAAAAGUGGCACCGAGUCGGUGC (SEQ ID NO:14),

wherein X₁₇₋₂₀ is a sequence complementary to the complementary strand of 17-20 consecutive nucleotides of a target sequence, preferably a target sequence immediately 5' of a protospacer adjacent motif (PAM), and X_{N} is any sequence that does not interfere with the binding of the ribonucleic acid to Cas9. Although a sequence of X₁₇₋₂₀ is exemplified herein with the *S. pyogenes* Cas9 system, longer sequences can also be used, e.g., as appropriate for other systems.

In some aspects of the disclosure, the DNA molecule also includes a sequence encoding a functional Csy4 enzyme.

Also provided herein are vectors comprising the DNA molecules described herein, e.g., optionally comprising a sequence encoding a functional Csy4 enzyme. Also provided herein are the multiplex transcripts produced by the DNA molecules, e.g., intact RNAs that have not yet been cleaved with Csy4.

In yet another aspect of the disclosure, provided herein are methods for producing a plurality of guide RNAs in a cell. The methods include expressing the DNA molecules described herein in the cell.

In some aspects of the disclosure, the cell is a mammalian cell, and the cell also expresses an exogenous functional Csy4 enzyme sequence, or the method further comprises administering a functional Csy4 enzyme or nucleic acid encoding a functional Csy4 enzyme.

In another aspect of the disclosure the invention provides methods for altering expression of one or a plurality of target genes in a cell. The methods include expressing a DNA molecule as described herein, e.g., a DNA molecule comprising a plurality of sequences encoding guide RNAs (gRNAs), wherein each gRNA comprises a variable sequence that is complementary to at least 17-20 nts of the one or more target genes, and each gRNA is flanked by at least one Csy4 cleavage sequence comprising or consisting of the sequence GTTCACTGCCGTATAGGCAG (SEQ ID NO:1) or GTTCACTGCCGTATAGGCAGCTAAGAAA (SEQ ID NO:2).

In the present methods and compositions, the gRNA can be either a single guide RNA comprising a fused tracrRNA and crRNA, as described herein, or can include just a crRNA, and the tracrRNA can be expressed from the same or a different DNA molecule. Thus in some embodiments the DNA molecules described herein also include a sequence encoding a tracrRNA. In some embodiments, the methods include expressing in the cells a separate tracrRNA, e.g., contacting the cells with a vector or DNA molecule that expresses a tracrRNA.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. In case of conflict, the present specification, including definitions, will control.

Other features and advantages of the invention will be apparent from the following detailed description and figures, and from the claims.

### DESCRIPTION OF DRAWINGS

Figure 1 is a schematic illustrating constructs used in initial multiplex experiments, as follows:
   1 + 2: direct repeat crRNA array and Cas9, with separate tracrRNA
   3 + 4: short crRNA array separated by Csy4 sites with Csy4, Cas9, and separate tracrRNA
   5 + 6: full-length chimeric gRNAs separated by Csy4 site.
   7: nls-FLAG tagged Cas9
Figure 2 is a bar graph showing the results of experiments in cells expressing the constructs shown in Figure 1. The Csy4 site + full gRNA (constructs 5 and 6) was the most efficient multiplex framework.
Figure 3 is a schematic overview and comparison of exemplary standard and multiplex Csy4-based gRNA frameworks, and the transcripts they produce. Note that Csy4 enables the use of RNA Pol II promoters (e.g., CAG) as an alternative to U6, an RNA Pol III promoter.
Figure 4 is a bar graph showing that Csy4 cleaves a truncated recognition site producing gRNAs with higher activity in human cells. Processing of the truncated site also leaves a clean 5' end, effectively removing the 5' G restriction on gRNA target sequences imposed by the U6 promoter.
Figures 5A-C are sequences showing evidence of 2-target multiplex editing in single human cells. Individual deletions are observed at intended site 2 or 3. Multiple deletions on the same sequence are observed for sites 2 and 3. Deletions spanning sites 2 and 3 are also observed.
Figure 6 is a schematic showing successful multiplex expression of three gRNAs using the Csy4-based system.
Figure 7 is a bar graph showing gRNAs excised by Csy4 from RNA Pol II-transcribed mRNA can efficiently recruit Cas9 nuclease to specific targets in human cells. In these experiments, gRNAs were expressed in longer mRNA transcripts made from the RNA Pol II CAG promoter.

### DETAILED DESCRIPTION

The invention is defined in the claims. One potential advantage of the Cas9 system is the capability to recruit either nuclease activity or heterologous effector domains to more than one genomic locus or target site in a cell. However, such multiplex applications require the ability to efficiently express more than one gRNA in a cell. For mammalian cells, RNA polymerase III promoters (e.g., U6 promoter) have been used to express single short gRNAs. Previous attempts to express multiple gRNA components in human cells from a single transcript have not proven to be efficient.

Additional desirable capabilities for the Cas9 system would be to create inducible versions of the components and to enable tissue-specific expression of the components. RNA polymerase II promoters that are inducible and/or tissue-specific have been previously described. However, although Cas9 or dCas9 proteins could be expressed from such RNA pol II promoters, short, defined gRNAs cannot be expressed in this way as the start and stop sites of transcription from RNA pol II are imprecise. Indeed, to date, all gRNAs have been expressed from RNA polymerase III promoters, which are ideally suited for expression of short RNAs.

As demonstrated herein, Csy4, an endoribonuclease that recognizes a short RNA hairpin sequence, can be used to cleave out multiple functional gRNAs encoded on a single longer RNA transcript (produced from an RNA pol III promoter) cassette in which the individual gRNAs are separated by Csy4 cleavage sites. Functional gRNAs can be successfully cleaved from longer RNA transcripts expressed from an RNA pol II promoter.

### gRNA/Csy4 Multimeric Cassettes

Thus described herein are DNA molecules that encode longer RNA transcripts, referred to herein as multimeric cassettes, which include two or more gRNA sequences, wherein each gRNA is flanked by a Csy4 cleavage sequence. The DNA molecules can also include a promoter, and can optionally include one or more other transcription regulatory sequences, e.g., enhancers, silencers, insulators, and polyA sequences. See, e.g., Xu et al., Gene. 2001 Jul 11;272(1-2):149-56.

### Promoters

A number of promoters are known in the art that can be used in the present methods. In some embodiments, the promoter is a PolII or Pol III promoter, preferably a Pol II promoter. Various Pol II promoters have been described and can be used in the present compositions and methods, including the CAG promoter (see, e.g., Alexopoulou et al., BMC Cell Biology 9: 2, 2008; Miyazaki et al., Gene 79 (2): 269-77 (1989); Niwa et al., Gene 108 (2): 193-9 (1991); additional promoters include the EF1A, CAGGS, PGK, UbiC and CMV promoters, as well as tissue-specific promoters such as B29, Desmin, Endoglin, FLT-1, GFPA, SYN1, among others; sequences of numerous promoters are known in the art. For example, the CMV and PGK promoters can be amplified from pSicoR and pSicoR PGK respectively (Ventura et al., Proc Natl Acad Sci U S A 101: 10380-10385 (2004)), the UbiC promoter can be amplified from pDSL hpUGIH (ATCC), the CAGGS promoter can be amplified from pCAGGS (BCCM), and the EF1A promoter can be amplified from the pEF6 vector (Invitrogen). The Pol II core promoter is described in Butler and Kadonaga, Genes & Dev. 16: 2583-2592 (2002). Cleavage of the gRNAs out of a larger transcript driven by Pol II expression enables one to produce gRNAs that have any nucleotide at the 5'-most position (standard expression from a U6 or other RNA polymerase III promoter places restrictions on the identity of this nucleotide).

In some embodiments, a tissue-specific promoter is used, and a short, defined gRNA sequence can be processed out of the RNA-Pol II transcript.

A number of Pol III promoters are known in the art, including the U6 small nuclear (sn) RNA promoter, 7SK promoter, and the HI promoter. See, e.g., Ro et al., BioTechniques, 38(4):625-627 (2005).

### Guide RNAs

Cas9 nuclease can be guided to specific genomic targets of at least 17-20 nts bearing an additional proximal protospacer adjacent motif (PAM) of sequence NGG by using a single gRNA bearing at least 17-20 nts at its 5' end that are complementary to the genomic DNA target site.

Thus, the compositions described herein can include a sequence encoding a single guide RNA (sgRNRA) comprising a crRNA fused to a normally trans-encoded tracrRNA, e.g., a single Cas9 guide RNA as described in Mali et al., Science 2013 Feb 15; 339(6121):823-6, with a sequence at the 5' end that is complementary to 17-20 nucleotides (nts) of a target sequence immediately 5' of a protospacer adjacent motif (PAM), e.g., NGG, NAG, or NNGG.

Methods of designing and expressing guide RNAs are known in the art. Guide RNAs generally speaking come in two different systems: 1) System 1 uses separate crRNA and tracrRNAs that function together to guide cleavage by Cas9; and 2) System 2 uses a chimeric crRNA-tracrRNA hybrid that combines the two separate guide RNAs in a single system (Jinek et al. 2012). The tracr-RNA can be variably truncated and a range of lengths has been shown to function in both the separate system (system 1) and the chimeric gRNA system (system 2). See, e.g., Jinek et al., Science 2012; 337:816-821; Mali et al., Science. 2013 Feb 15;339(6121):823-6; Cong et al., Science. 2013 Feb 15;339(6121):819-23; and Hwang and Fu et al., Nat Biotechnol. 2013 Mar;31(3):227-9; Jinek et al., Elife 2, e00471 (2013)). For System 2, generally the longer length chimeric gRNAs have shown greater on-target activity but the relative specificities of the various length gRNAs currently remain undefined and therefore it may be desirable in certain instances to use shorter gRNAs. In some aspects of the disclosure, the gRNAs are complementary to a region that is within about 100-800 bp upstream of the transcription start site, e.g., is within about 500bp upstream of the transcription start site, includes the transcription start site, or within about 100-800 bp, e.g., within about 500 bp, downstream of the transcription start site. In some aspects of the disclosure, vectors (e.g., plasmids) encoding more than one gRNA are used, e.g., plasmids encoding, 2, 3, 4, 5, or more gRNAs directed to different sites in the same region of the target gene. Additional guide RNAs, and methods of increasing the specificity of genome editing, are described in Provisional Patent Application Serial No. 61/838,178, entitled INCREASING SPECIFICITY FOR RNA-GUIDED GENOME EDITING

In some aspects of the disclosure, the gRNA comprises or consists of the sequence:
(X₁₇₋₂₀) GUUUUAGAGCUAUGCUGUUUUG(X_{N}) (SEQ ID NO:4);
(X₁₇₋₂₀)GUUUUAGAGCUA (SEQ ID NO:5);
(X₁₇₋₂₀) GUUUUAGAGCUAUGCUGUUUUG (SEQ ID NO:6);
(X₁₇₋₂₀)GUUUUAGAGCUAUGCU (SEQ ID NO:7);
(X₁₇₋₂₀)GUUUUAGAGCUAGAAAUAGCAAGUUAAAAUAAGGCUAGUCCG (X_{N}) (SEQ ID NO:8);

(X17-20)GUUUUAGAGCUAUGCUGAAAAGCAUAGCAAGUUAAAAUAAGGCUAGUC
CGUUAUC(XN) (SEQ ID NO:9);

(X17-20)GUUUUAGAGCUAUGCUGUUUUGGAAACAAAACAGCAUAGCAAGUUAAA
AUAAGGCUAGUCCGUUAUC(XN) (SEQ ID NO:10);

(X17-20)GUUUUAGAGCUAGAAAUAGCAAGUUAAAAUAAGGCUAGUCCGUUAUCA
ACUUGAAAAAGUGGCACCGAGUCGGUGC(XN) (SEQ ID NO:11),

(X17-20)GUUUAAGAGCUAGAAAUAGCAAGUUUAAAUAAGGCUAGUCCGUUAUCA
ACUUGAAAAAGUGGCACCGAGUCGGUGC(SEQ ID NO:12);

(X17-20)GUUUUAGAGCUAUGCUGGAAACAGCAUAGCAAGUUUAAAUAAGGCUAG
UCCGUUAUCAACUUGAAAAAGUGGCACCGAGUCGGUGC (SEQ ID NO:13); or

(X17-20)GUUUAAGAGCUAUGCUGGAAACAGCAUAGCAAGUUUAAAUAAGGCUAG
UCCGUUAUCAACUUGAAAAAGUGGCACCGAGUCGGUGC (SEQ ID NO:14),

wherein X₁₇₋₂₀ is a sequence complementary to the complementary strand of at least 17-20 consecutive nucleotides of a target sequence (though in some aspects of the disclosure this complementarity region may be longer than 20 nts, e.g., 21, 22, 23, 24, 25, 26, 27, 28, 29, or 30 or more nts, e.g., 17-30 nts), preferably a target sequence immediately 5' of a protospacer adjacent motif (PAM), e.g., NGG, NAG, or NNGG. X_{N} is any sequence, wherein N (in the RNA) can be 0-300 or 0-200, e.g., 0-100, 0-50, or 0-20, that does not interfere with the binding of the ribonucleic acid to Cas9. In some aspects of the disclosure the RNA includes one or more U, e.g., 1 to 8 or more Us (e.g., U, UU, UUU, UUUU, UUUUU, UUUUUU, UUUUUUU, UUUUUUUU) at the 3' end of the molecule, as a result of the optional presence of one or more Ts used as a termination signal to terminate RNA PolIII transcription. In some aspects of the disclosure the RNA includes one or more, e.g., up to 3, e.g., one, two, or three, or more additional nucleotides at the 5' end of the RNA molecule that is not complementary to the target sequence. Optionally, one or more of the RNA nucleotides is modified, e.g., locked (2'-O-4'-C methylene bridge), is 5'-methylcytidine, is 2'-O-methyl-pseudouridine, or in which the ribose phosphate backbone has been replaced by a polyamide chain, e.g., one or more of the nucleotides within the sequence X₁₇₋₂₀, one or more of the nucleotides within the sequence X_{N}, or one or more of the nucleotides within any sequence of the gRNA

For example, in some aspects of the disclosure the chimeric guide RNAs described in Jinek et al. (Science. 337(6096):816-21 (2012)) can be used, e.g., 
(X17-20)GUUUUAGAGCUAGAAAUAGCAAGUUAAAAUAAGGCUAGUCCG (SEQ ID
NO:8); (X17-20)GUUUUAGAGCUAGAAAUAGCAAGUUAAAAUAAGG (SEQ ID NO:9);
 in some embodiments, the sgRNA bearing a 5'-terminal 17-20-nucleotide sequence complementary to the target DNA sequence, and a 42-nucleotide 3'-terminal stem loop structure required for Cas9 binding described in Jinek et al., Elife. 2:e00471 (2013), e.g., (X₁₇₋₂₀)GUUUUAGAGCUAGAAAUAGCAAGUUAAAAUAAGGCUAGUCCG (SEQ ID NO:8) are used.

In some aspects of the disclosure, the guide RNA includes one or more Adenine (A) or Uracil (U) nucleotides on the 3' end.

Although the examples described herein utilize a single gRNA, the methods can also be used with dual gRNAs (e.g., the crRNA and tracrRNA found in naturally occurring systems). In this case, a single tracrRNA would be used in conjunction with multiple different crRNAs expressed using the present system, e.g., the following (note that for RNAs, T's are understood herein to be U's):
crRNA sequence: X₁₇₋₂₀-GTTTTAGAGCTAGAAA (SEQ ID NO: 15)
tracrRNA sequence: TAGCAAGTTAAAATAAGGCTAGTCCGTTATCAACTTGAAAAAGTGGCACCGAGT CGGTGC (SEQ ID NO: 16). In this case, the crRNA is used as the guide RNA in the methods and molecules described herein, and the tracrRNA can be expressed from the same or a different DNA molecule.

Furthermore, although guide RNAs having a sequence of 17-20 nucleotides of complementarity are exemplified herein, in some aspects of the disclosure longer sequences can be used, e.g., 21, 22, 23, 24, 25, 26, 27, 28, 29, or 30 or more nts, e.g., 17-30 nts in place of 17-20 nts.

### Csy4 Cleavage Sequences

In the methods and compositions described herein, a Csy4 cleavage sequence is inserted into the DNA molecule such that each guide RNA is flanked by a cleavage sequence, with one or at least one cleavage sequence between each guide RNA. Exemplary Cys4 cleavage sequences include GTTCACTGCCGTATAGGCAG (truncated 20 nt) (SEQ ID NO: 1) and GTTCACTGCCGTATAGGCAGCTAAGAAA (full 28 nt) (SEQ ID NO:2). As demonstrated herein, use of the truncated Csy4 cleavage site (SEQ ID NO:1) is more efficient in human cells than use of the standard site. To the best of the present inventors' knowledge, this is the first demonstration of Csy4 activity being utilized in human cells.

### Functional Csy4 Enzyme sequences

In the methods described herein, a functional Csy4 enzyme that is capable of cleaving the transcripts at the Csy4 cleavage sites, is also expressed in the cell.

Exemplary Csy4 sequences from Csy4 homologues from Pseudomonas aeruginosa UCBPP-PA14 (Pa14), Yersinia pestis AAM85295 (Yp), Escherichia coli UTI89 (Ec89), Dichelobacter nodosus VCS1703A (Dn), Acinetobacter baumannii AB0057 (Ab), Moritella sp. PE36 (MP1, MP01), Shewanella sp. W3-18-1 (SW), Pasteurella multocida subsp. multocida Pm70 (Pm), Pectobacterium wasabiae (Pw), and Dickeya dadantii Ech703 (Dd) are set forth in Fig. S6 of Haurwitz et al., Science 329(5997): 1355-1358 (2010). In preferred embodiments, the Csy4 is from *Pseudomonas aeruginosa.*

In some embodiments, the Csy4 is also used to covalently link heterologous effector domains to the gRNAs. Csy4 is believed to be a single-turnover enzyme and remains bound to its target hairpin sequence after cleavage (Sternberg et al., RNA. 2012 Apr;18(4):661-72). Csy4 is thus expected to remain bound to the 3' end of each cleaved gRNA. Since as demonstrated herein the cleaved gRNAs appear to be functional in human cells, the presence of this Csy4 protein on the 3' end of the gRNA does not appear to affect the ability of the gRNA to complex with and direct Cas9 activity. Thus it is presumed that these gRNA-Csy4 fusions would also be able to direct Cas9 mutants that bear mutations that inactivate its catalytic nuclease activity (dCas9 proteins). Therefore, one could fuse heterologous functional domains (HFD) to Csy4 (Csy4-HFD), and a dCas9:sgRNA:Csy4-HFD complex could then direct such domains to a specific genomic locus. Examples of such HFD could include other nuclease domains such as that from Fokl, transcriptional activator or repressor domains, or other domains that modify histones or DNA methylation status.

The Csy4-HFD are created by fusing a heterologous functional domain (e.g., a transcriptional activation domain, e.g., from VP64 or NF-κB p65), to the N-terminus or C-terminus of a Csy4 protein, with or without an intervening linker, e.g., a linker of about 5-20 or 13-18 nucleotides. The transcriptional activation domains can be fused on the N or C terminus of the Csy4. In addition to transcriptional activation domains, other heterologous functional domains (e.g., transcriptional repressors (e.g., KRAB, SID, and others) or silencers such as Heterochromatin Protein 1 (HP1, also known as swi6), e.g., HP1α or HP1β; proteins or peptides that could recruit long non-coding RNAs (IncRNAs) fused to a fixed RNA binding sequence such as those bound by the MS2 coat protein, endoribonuclease Csy4, or the lambda N protein; enzymes that modify the methylation state of DNA (e.g., DNA methyltransferase (DNMT) or TET proteins); or enzymes that modify histone subunits (e.g., histone acetyltransferases (HAT), histone deacetylases (HDAC), histone methyltransferases (e.g., for methylation of lysine or arginine residues) or histone demethylases(e.g., for demethylation of lysine or arginine residues)) as are known in the art can also be used. A number of sequences for such domains are known in the art, e.g., a domain that catalyzes hydroxylation of methylated cytosines in DNA. Exemplary proteins include the Ten-Eleven-Translocation (TET)1-3 family, enzymes that converts 5-methylcytosine (5-mC) to 5-hydroxymethylcytosine (5-hmC) in DNA. See, e.g., WO/2014/144761.

Sequences for human TET1-3 are known in the art and are shown in the following table:

| | **GenBank Accession Nos.** | |
|---|---|---|
| Gene | Amino Acid | Nucleic Acid |
| TET1 | NP_085128.2 | NM_030625.2 |
| TET2* | NP_001120680.1 (var 1) | NM_001127208.2 |
| | NP_060098.3 (var 2) | NM_017628.4 |
| TET3 | NP_659430.1 | NM_144993.1 |

| | | |
|---|---|---|
| * Variant (1) represents the longer transcript and encodes the longer isoform (a). Variant (2) differs in the 5' UTR and in the 3' UTR and coding sequence compared to variant 1. The resulting isoform (b) is shorter and has a distinct C-terminus compared to isoform a. | | |

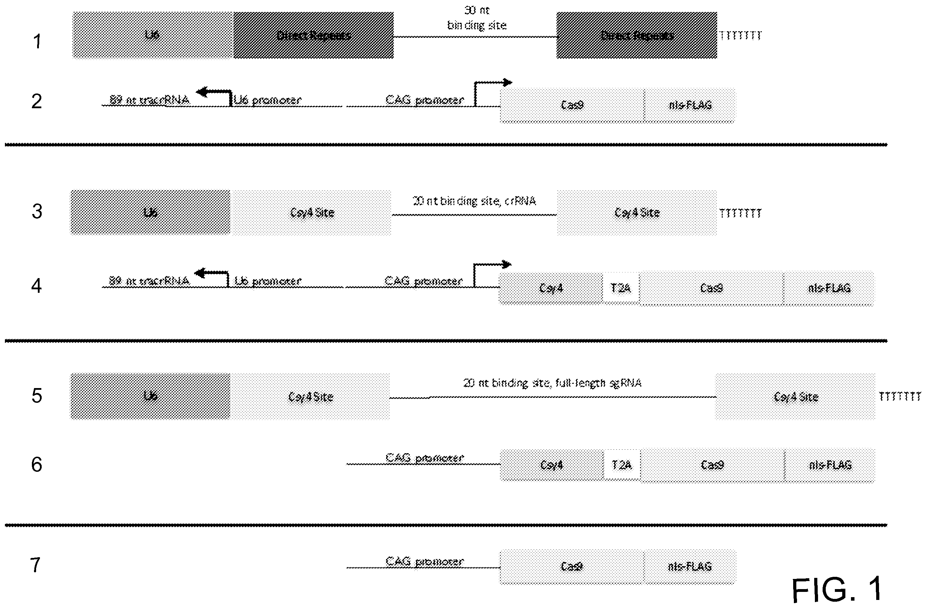

In some embodiments, all or part of the full-length sequence of the catalytic domain can be included, e.g., a catalytic module comprising the cysteine-rich extension and the 2OGFeDO domain encoded by 7 highly conserved exons, e.g., the Tet1 catalytic domain comprising amino acids 1580-2052, Tet2 comprising amino acids 1290-1905 and Tet3 comprising amino acids 966-1678. See, e.g., Fig. 1 of Iyer et al., Cell Cycle. 2009 Jun 1;8(11): 1698-710. Epub 2009 Jun 27, for an alignment illustrating the key catalytic residues in all three Tet proteins, and the supplementary materials thereof (available at ftp site ftp.ncbi.nih.gov/pub/aravind/DONS/supplementary_material_DONS.html) for full length sequences (see, e.g., seq 2c); in some embodiments, the sequence includes amino acids 1418-2136 of Tet1 or the corresponding region in Tet2/3.

Other catalytic modules can be, e.g., from the proteins identified in Iyer et al., 2009.

In some embodiments, the fusion proteins include a linker between the Csy4 and the heterologous functional domains. Linkers that can be used in these fusion proteins (or between fusion proteins in a concatenated structure) can include any sequence that does not interfere with the function of the fusion proteins. In preferred embodiments, the linkers are short, e.g., 2-20 amino acids, and are typically flexible (i.e., comprising amino acids with a high degree of freedom such as glycine, alanine, and serine). In some embodiments, the linker comprises one or more units consisting of GGGS (SEQ ID NO:3) or GGGGS (SEQ ID NO: 17), e.g., two, three, four, or more repeats of the GGGS (SEQ ID NO:3) or GGGGS (SEQ ID NO: 17) unit. Other linker sequences can also be used, e.g., GGS, GGSG (SEQ ID NO:22), SGSETPGTSESA (SEQ ID NO:23), SGSETPGTSESATPES (SEQ ID NO:24), or SGSETPGTSESATPEGGSGGS (SEQ ID NO:25).

### Cas9

In the methods described herein, Cas9 is also expressed in the cells. A number of bacteria express Cas9 protein variants. The Cas9 from *Streptococcus pyogenes* is presently the most commonly used; some of the other Cas9 proteins have high levels of sequence identity with the *S. pyogenes* Cas9 and use the same guide RNAs. Others are more diverse, use different gRNAs, and recognize different PAM sequences as well (the 2-5 nucleotide sequence specified by the protein which is adjacent to the sequence specified by the RNA). Chylinski et al. classified Cas9 proteins from a large group of bacteria (RNA Biology 10:5, 1-12; 2013), and a large number of Cas9 proteins are listed in supplementary figure 1 and supplementary table 1 thereof. The constructs and methods described herein can include the use of any of those Cas9 proteins, and their corresponding guide RNAs or other guide RNAs that are compatible. The Cas9 from *Streptococcus thermophilus* LMD-9 CRISPR1 system has also been shown to function in human cells in Cong et al (Science 339, 819 (2013)). Additionally, Jinek et al. showed *in vitro* that Cas9 orthologs from S. *thermophilus* and *L. innocua,* (but not from *N. meningitidis* or *C. jejuni,* which likely use a different guide RNA), can be guided by a dual *S. pyogenes* gRNA to cleave target plasmid DNA, albeit with slightly decreased efficiency.

In some embodiments, the present system utilizes the Cas9 protein from *S. pyogenes,* either as encoded in bacteria or codon-optimized for expression in mammalian cells. An exemplary sequence of *Streptococcus pyogenes* Cas9 (residues 1-1368) fused to an HA epitope (amino acid sequence DAYPYDVPDYASL (SEQ ID NO: 18)) and a nuclear localization signal (amino acid sequence PKKKRKVEDPKKKRKVD (SEQ ID NO:19)) is as follows: 
MDKKYSIGLDIGTNSVGWAVITDEYKVPSKKFKVLGNTDRHSIKKNLIGALLFDSGETAEATRLKRTARRRYTRRKNR
ICYLQEIFSNEMAKVDDSFFHRLEESFLVEEDKKHERHPIFGNIVDEVAYHEKYPTIYHLRKKLVDSTDKADLRLIYL
ALAHMIKFRGHFLIEGDLNPDNSDVDKLFIQLVQTYNQLFEENPINASGVDAKAILSARLSKSRRLENLIAQLPGEKKNG
LFGNLIALSLGLTPNFKSNFDLAEDAKLQLSKDTYDDDLDNLLAQIGDQYADLFLAAKNLSDAILLSDILRVNTEITKAP
LSASMIKRYDEHHQDLTLLKALVRQQLPEKYKEIFFDQSKNGYAGYIDGGASQEEFYKFIKPILEKMDGTEELLVKLNRE
DLLRKQRTFDNGSIPHQIHLGELHAILRRQEDFYPFLKDNREKIEKILTFRIPYYVGPLARGNSRFAWMTRKSEETITPW
NFEEVVDKGASAQSFIERMTNFDKNLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENEDILEDIVLTLTLFEDREMIEERL
KTYAHLFDDKVMKQLKRRRYTGWGRLSRKLINGIRDKQSGKTILDFLKSDGFANRNFMQLIHDDSLTFKEDIQKAQVSGQ
GDSLHEHIANLAGSPAIKKGILQTVKVVDELVKVMGRHKPENIVIEMARENQTTQKGQKNSRERMKRIEEGIKELGSQIL
KEHPVENTQLQNEKLYLYYLQNGRDMYVDQELDINRLSDYDVDHIVPQSFLKDDSIDNKVLTRSDKNRGKSDNVPSEEVV
KKMKNYWRQLLNAKLITQRKFDNLTKAERGGLSELDKAGFIKRQLVETRQITKHVAQILDSRMNTKYDENDKLIREVKVI
TLKSKLVSDFRKDFQFYKVREINNYHHAHDAYLNAVVGTALIKKYPKLESEFVYGDYKVYDVRKMIAKSEQEIGKATAKY
FFYSNIMNFFKTEITLANGEIRKRPLIETNGETGEIVWDKGRDFATVRKVLSMPQVNIVKKTEVQTGGFSKESILPKRNS
DKLIARKKDWDPKKYGGFDSPTVAYSVLVVAKVEKGKSKKLKSVKELLGITIMERSSFEKNPIDFLEAKGYKEVKKDLII
KLPKYSLFELENGRKRMLASAGELQKGNELALPSKYVNFLYLASHYEKLKGSPEDNEQKQLFVEQHKHYLDEIIEQISEF
SKRVILADANLDKVLSAYNKHRDKPIREQAENIIHLFTLTNLGAPAAFKYFDTTIDRKRYTSTKEVLDATLIHQSITGLY
ETRIDLSQLGGDAYPYDVPDYASLGSGSPKKKRKVEDPKKKRKVD     (SEQ ID NO:20)
 See Jinek et al, 2013, *supra.*

In some embodiment, a Cas9 sequence is used that contains either of the D10A and H840A mutations to render the nuclease a nickase, or both the D10A and H840A mutations to render the nuclease portion of the protein catalytically inactive. The sequence of a catalytically inactive *S. pyogenes* Cas9 (dCas9) that can be used in the methods and compositions described herein is as follows; the mutations are in bold and underlined. 
10 20 30 40 50 60
MDKKYSIGLA IGTNSVGWAV ITDEYKVPSK KFKVLGNTDR HSIKKNLIGA LLFDSGETAE

70 80 90 100 110 120
ATRLKRTARR RYTRRKNRIC YLQEIFSNEM AKVDDSFFHR LEESFLVEED KKHERHPIFG

130 140 150 160 170 180
NIVDEVAYHE KYPTIYHLRK KLVDSTDKAD LRLIYLALAH MIKFRGHFLI EGDLNPDNSD

190 200 210 220 230 240
VDKLFIQLVQ TYNQLFEENP INASGVDAKA ILSARLSKSR RLENLIAQLP GEKKNGLFGN

250 260 270 280 290 300
LIALSLGLTP NFKSNFDLAE DAKLQLSKDT YDDDLDNLLA QIGDQYADLF LAAKNLSDAI

310 320 330 340 350 360
LLSDILRVNT EITKAPLSAS MIKRYDEHHQ DLTLLKALVR QQLPEKYKEI FFDQSKNGYA

370 380 390 400 410 420
GYIDGGASQE EFYKFIKPIL EKMDGTEELL VKLNREDLLR KQRTFDNGSI PHQIHLGELH
 
430 440 450 460 470 480
AILRRQEDFY PFLKDNREKI EKILTFRIPY YVGPLARGNS RFAWMTRKSE ETITPWNFEE

490 500 510 520 530 540
VVDKGASAQS FIERMTNFDK NLPNEKVLPK HSLLYEYFTV YNELTKVKYV TEGMRKPAFL

550 560 570 580 590 600
SGEQKKAIVD LLFKTNRKVT VKQLKEDYFK KIECFDSVEI SGVEDRFNAS LGTYHDLLKI

610 620 630 640 650 660
IKDKDFLDNE ENEDILEDIV LTLTLFEDRE MIEERLKTYA HLFDDKVMKQ LKRRRYTGWG

670 680 690 700 710 720
RLSRKLINGI RDKQSGKTIL DFLKSDGFAN RNFMQLIHDD SLTFKEDIQK AQVSGQGDSL

730 740 750 760 770 780
HEHIANLAGS PAIKKGILQT VKVVDELVKV MGRHKPENIV IEMARENQTT QKGQKNSRER

790 800 810 820 830 840
MKRIEEGIKE LGSQILKEHP VENTQLQNEK LYLYYLQNGR DMYVDQELDI NRLSDYDVDA

850 860 870 880 890 900
IVPQSFLKDD SIDNKVLTRS DKNRGKSDNV PSEEVVKKMK NYWRQLLNAK LITQRKFDNL

910 920 930 940 950 960
TKAERGGLSE LDKAGFIKRQ LVETRQITKH VAQILDSRMN TKYDENDKLI REVKVITLKS

970 980 990 1000 1010 1020
KLVSDFRKDF QFYKVREINN YHHAHDAYLN AVVGTALIKK YPKLESEFVY GDYKVYDVRK

1030 1040 1050 1060 1070 1080
MIAKSEQEIG KATAKYFFYS NIMNFFKTEI TLANGEIRKR PLIETNGETG EIVWDKGRDF

1090 1100 1110 1120 1130 1140
ATVRKVLSMP QVNIVKKTEV QTGGFSKESI LPKRNSDKLI ARKKDWDPKK YGGFDSPTVA

1150 1160 1170 1180 1190 1200
YSVLVVAKVE KGKSKKLKSV KELLGITIME RSSFEKNPID FLEAKGYKEV KKDLIIKLPK

1210 1220 1230 1240 1250 1260
YSLFELENGR KRMLASAGEL QKGNELALPS KYVNFLYLAS HYEKLKGSPE DNEQKQLFVE

1270 1280 1290 1300 1310 1320
QHKHYLDEII EQISEFSKRV ILADANLDKV LSAYNKHRDK PIREQAENII HLFTLTNLGA

1330 1340 1350 1360
PAAFKYFDTT IDRKRYTSTK EVLDATLIHQ SITGLYETRI DLSQLGGD (SEQ ID NO:21)
 See, e.g., Mali et al., 2013, *supra*; and Jinek et al., 2012, *supra.* Alternatively, the Cas9 can be a dCas9-heterofunctional domain fusion (dCas9-HFD) as described in U.S. Provisional Patent Application entitled RNA-GUIDED TARGETING OF GENETIC AND EPIGENOMIC REGULATORY PROTEINS TO SPECIFIC GENOMIC LOCI, filed on June 21, 2013 and assigned serial no. 61/838,148, and in PCT/US2014/027335.

The Cas9 can be expressed from an expression vector, as described herein, e.g., an extrachromosomal plasmid or viral vector comprising a sequence encoding Cas9, e.g., a Cas9 cDNA or gene; can be expressed from an exogenous Cas9 cDNA or gene that has integrated into the genome of the cell; an mRNA encoding Cas9; the actual Cas9 protein itself; or, in the case of non-mammalian cells, can be an exogenous Cas9.

### Expression Systems

Nucleic acid molecules comprising expression vectors can be used, e.g., for in vivo or in vitro expression of the Csy4/guide RNA constructs described herein. Vectors for expressing multiple gRNAs (potentially in an inducible or tissue-/cell-type specific fashion) can be used for research and therapeutic applications.

In order to use the fusion proteins and multimeric guide RNA cassettes described herein, it may be desirable to express them from a nucleic acid that encodes them. This can be performed in a variety of ways. For example, a nucleic acid encoding a guide RNA cassette or Csy4 or Cas9 protein can be cloned into an intermediate vector for transformation into prokaryotic or eukaryotic cells for replication and/or expression. Intermediate vectors are typically prokaryote vectors, e.g., plasmids, or shuttle vectors, or insect vectors, for storage or manipulation of the nucleic acid encoding the fusion protein or for production of the fusion protein. The nucleic acid encoding the guide RNA or fusion protein can also be cloned into an expression vector, for administration to a plant cell, animal cell, preferably a mammalian cell or a human cell, fungal cell, bacterial cell, or protozoan cell.

To obtain expression, a sequence encoding a guide RNA or fusion protein is typically subcloned into an expression vector that contains a promoter to direct transcription. Suitable bacterial and eukaryotic promoters are well known in the art and described, e.g., in Sambrook et al., Molecular Cloning, A Laboratory Manual (3d ed. 2001); Kriegler, Gene Transfer and Expression: A Laboratory Manual (1990); and Current Protocols in Molecular Biology (Ausubel et al., eds., 2010). Bacterial expression systems for expressing the engineered protein are available in, e.g., *E. coli, Bacillus* sp., and *Salmonella* (Palva et al., 1983, Gene 22:229-235). Kits for such expression systems are commercially available. Eukaryotic expression systems for mammalian cells, yeast, and insect cells are well known in the art and are also commercially available.

A number of suitable vectors are known in the art, e.g., viral vectors including recombinant retroviruses, lentivirus, adenovirus, adeno-associated virus, and herpes simplex virus 1, adenovirus-derived vectors, or recombinant bacterial or eukaryotic plasmids. For example, the expression construct can include: a coding region; a promoter sequence, e.g., a promoter sequence that restricts expression to a selected cell type, a conditional promoter, or a strong general promoter; an enhancer sequence; untranslated regulatory sequences, e.g., a 5'untranslated region (UTR), a 3'UTR; a polyadenylation site; and/or an insulator sequence. Such sequences are known in the art, and the skilled artisan would be able to select suitable sequences. See, e.g., Current Protocols in Molecular Biology, Ausubel, F.M. et al. (eds.) Greene Publishing Associates, (1989), Sections 9.10-9.14 and other standard laboratory manuals. In some embodiments, expression can be restricted to a particular cell type, using a tissue-specific promoter as is known in the art.

As described above, the vectors for expressing the guide RNAs can include RNA Pol II or Pol III promoters to drive expression of the guide RNAs. These human promoters allow for expression of gRNAs in mammalian cells following plasmid transfection. Alternatively, a T7 promoter may be used, e.g., for in vitro transcription, and the RNA can be transcribed in vitro and purified. The promoter used to direct expression of the nucleic acid depends on the particular application. For example, a strong constitutive promoter is typically used for expression and purification of fusion proteins. In contrast, when the fusion protein is to be administered in vivo for gene regulation, either a constitutive or an inducible promoter can be used, depending on the particular use of the fusion protein. In addition, a preferred promoter for administration of the fusion protein can be a weak promoter, such as HSV TK or a promoter having similar activity. The promoter can also include elements that are responsive to transactivation, e.g., hypoxia response elements, Gal4 response elements, lac repressor response element, and small molecule control systems such as tetracycline-regulated systems and the RU-486 system (see, e.g., Gossen & Bujard, 1992, Proc. Natl. Acad. Sci. USA, 89:5547; Oligino et al., 1998, Gene Ther., 5:491-496; Wang et al., 1997, Gene Ther., 4:432-441; Neering et al., 1996, Blood, 88:1147-55; and Rendahl et al., 1998, Nat. Biotechnol., 16:757-761).

In addition to the promoter, the expression vector typically contains a transcription unit or expression cassette that contains all the additional elements required for the expression of the nucleic acid in host cells, either prokaryotic or eukaryotic. A typical expression cassette thus contains a promoter operably linked, e.g., to the nucleic acid sequence encoding the fusion protein, and any signals required, e.g., for efficient polyadenylation of the transcript, transcriptional termination, ribosome binding sites, or translation termination. Additional elements of the cassette may include, e.g., enhancers, and heterologous spliced intronic signals.

The particular expression vector used to transport the genetic information into the cell is selected with regard to the intended use of the fusion protein, e.g., expression in plants, animals, bacteria, fungus, protozoa, etc. Standard bacterial expression vectors include plasmids such as pBR322 based plasmids, pSKF, pET23D, and commercially available tag-fusion expression systems such as GST and LacZ. A preferred tag-fusion protein is the maltose binding protein (MBP). Such tag-fusion proteins can be used for purification of the engineered TALE repeat protein. Epitope tags can also be added to recombinant proteins to provide convenient methods of isolation, for monitoring expression, and for monitoring cellular and subcellular localization, e.g., c-myc or FLAG.

Expression vectors containing regulatory elements from eukaryotic viruses are often used in eukaryotic expression vectors, e.g., SV40 vectors, papilloma virus vectors, and vectors derived from Epstein-Barr virus. Other exemplary eukaryotic vectors include pMSG, pAV009/A+, pMT010/A+, pMAMneo-5, baculovirus pDSVE, and any other vector allowing expression of proteins under the direction of the SV40 early promoter, SV40 late promoter, metallothionein promoter, murine mammary tumor virus promoter, Rous sarcoma virus promoter, polyhedrin promoter, or other promoters shown effective for expression in eukaryotic cells.

Some expression systems have markers for selection of stably transfected cell lines such as thymidine kinase, hygromycin B phosphotransferase, and dihydrofolate reductase. High yield expression systems are also suitable, such as using a baculovirus vector in insect cells, with the fusion protein encoding sequence under the direction of the polyhedrin promoter or other strong baculovirus promoters.

The elements that are typically included in expression vectors also include a replicon that functions in *E. coli,* a gene encoding antibiotic resistance to permit selection of bacteria that harbor recombinant plasmids, and unique restriction sites in nonessential regions of the plasmid to allow insertion of recombinant sequences.

Standard transfection methods are used to produce bacterial, mammalian, yeast or insect cell lines that express large quantities of protein, which are then purified using standard techniques (see, e.g., Colley et al., 1989, J. Biol. Chem., 264:17619-22; Guide to Protein Purification, in Methods in Enzymology, vol. 182 (Deutscher, ed., 1990)). Transformation of eukaryotic and prokaryotic cells are performed according to standard techniques (see, e.g., Morrison, 1977, J. Bacteriol. 132:349-351; Clark-Curtiss & Curtiss, Methods in Enzymology 101:347-362 (Wu et al., eds, 1983).

Any of the known procedures for introducing foreign nucleotide sequences into host cells may be used. These include the use of calcium phosphate transfection, polybrene, protoplast fusion, electroporation, nucleofection, liposomes, microinjection, naked DNA, plasmid vectors, viral vectors, both episomal and integrative, and any of the other well-known methods for introducing cloned genomic DNA, cDNA, synthetic DNA or other foreign genetic material into a host cell (see, e.g., Sambrook et al., supra). It is only necessary that the particular genetic engineering procedure used be capable of successfully introducing at least one gene into the host cell capable of expressing the protein of choice.

In some embodiments, the Cas9 or Csy4 protein includes a nuclear localization domain which provides for the protein to be translocated to the nucleus. Several nuclear localization sequences (NLS) are known, and any suitable NLS can be used. For example, many NLSs have a plurality of basic amino acids, referred to as a bipartite basic repeats (reviewed in Garcia-Bustos et al, 1991, Biochim. Biophys. Acta, 1071:83-101). An NLS containing bipartite basic repeats can be placed in any portion of chimeric protein and results in the chimeric protein being localized inside the nucleus. In preferred embodiments a nuclear localization domain is incorporated into the final fusion protein, as the ultimate functions of the fusion proteins described herein will typically require the proteins to be localized in the nucleus. However, it may not be necessary to add a separate nuclear localization domain in cases where the protein has intrinsic nuclear translocation function.

The present invention includes the vectors and cells comprising the vectors.

### Libraries

Also provided herein are combinatorial libraries of gRNAs, e.g., in inducible, tissue-or cell-type specific multiplex vectors for research applications, e.g., for screening for potential drug targets or to define interactions at a genetic level.

### Methods of Use

The methods described can include expressing in a cell, or contacting the cell with, the multimeric cassettes as described herein, plus a nuclease that can be guided by the shortened gRNAs, e.g., a Cas9 nuclease as described above, and a Csy4 nuclease, as described above.

The described system is a useful and versatile tool for modifying the expression of multiple endogenous genes simultaneously, or for targeting multiple parts of a single gene. Current methods for achieving this require the use of a separate gRNA-encoding transcript for each site to be targeted. Separate gRNAs are not optimal for multiplex genome editing of cell populations as it is impossible to guarantee that each cell will express each gRNA; with multiple transcripts, cells get a complex and non-uniform random mixture of gRNAs. The present system, however, allows expression of multiple gRNAs from a single transcript, which allows targeting of multiple sites in the genome by expression of multiple gRNAs. Furthermore, with a single-transcript system, each cell should express all gRNAs with similar stoichiometry. This system could therefore easily be used to simultaneously alter expression of a large number of genes or to recruit multiple Cas9s or HFDs to a single gene, promoter, or enhancer. This capability will have broad utility, e.g., for basic biological research, where it can be used to study gene function and to manipulate the expression of multiple genes in a single pathway, and in synthetic biology, where it will enable researchers to create circuits in cell that are responsive to multiple input signals. The relative ease with which this technology can be implemented and adapted to multiplexing will make it a broadly useful technology with many wide-ranging applications.

The methods described herein include contacting cells with a nucleic acid encoding the multimeric gRNA cassettes described herein directed to one or more genes, and nucleic acids encoding Csy4 and Cas9, to thereby modulate expression of the one or more genes.

### EXAMPLES

The invention is further described in the following examples, which do not limit the scope of the invention described in the claims.

### Example 1. Multiplex Editing with CRISPR/Cas9

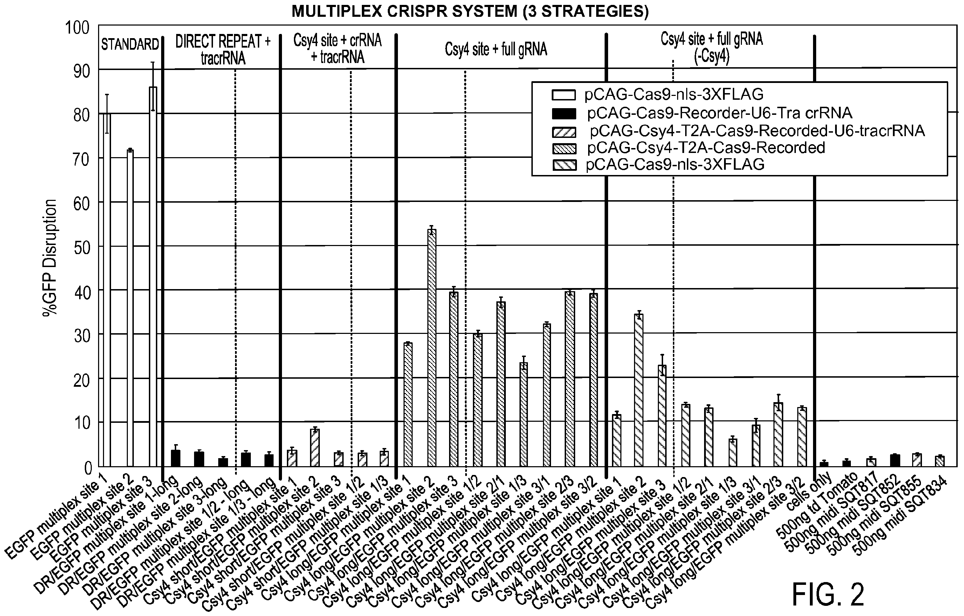

Three strategies were tried with the objective of making multiplex edits with CRISPR/Cas9 from arrays of either crRNAs or sgRNAs expressed from a single transcript, as follows:
1. direct-repeat flanked crRNA array and Cas9, with separate tracrRNA
2. short crRNA array separated by Csy4 sites, expressed with Csy4, Cas9, and separate tracrRNA
3. full-length single guide RNAs (sgRNAs) separated by Csy4 sites
Each set of constructs (illustrated in Figure 1) was tested for the ability to efficiently disrupt EGFP in a U2OS-EGFP disruption assay. The results are shown in Figure 2. Constructs designed using strategies 1 and 2 exhibited the lowest activity in the EGFP-disruption assay even for single targets; therefore further experiments (described below) focused on optimizing strategy 3.

### Example 2. Multiplex Expression of Highly Active CRISPR Guide RNAs from RNA Polymerase II and III Promoters in Mammalian Cells

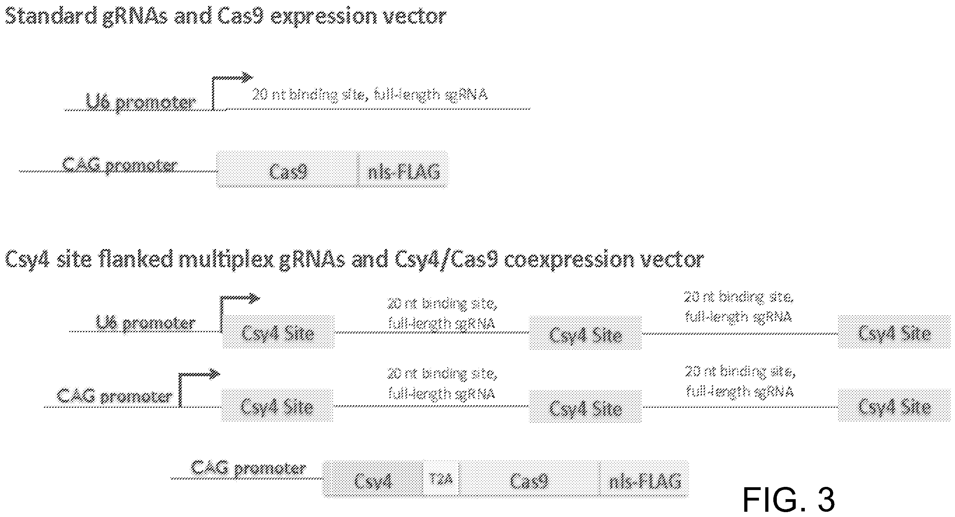

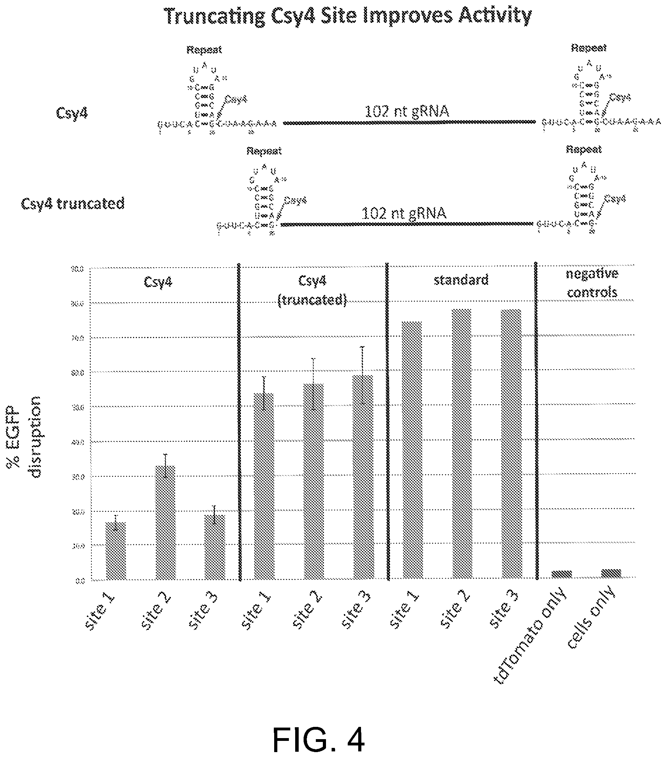

A schematic overview of an exemplary strategy for cleaving gRNAs out from longer transcripts using the Csy4 nuclease is shown in Figure 3. In initial experiments to demonstrate proof-of-concept, two versions of the Csy4-cleaved RNA hairpin site were tested for cleavage in human cells. To do this, gRNAs flanked by one of two Csy4 cleavage sites were expressed:
1. GTTCACTGCCGTATAGGCAGCTAAGAAA (full 28 nt) (SEQ ID NO:2)
2. GTTCACTGCCGTATAGGCAG (truncated 20 nt) (SEQ ID NO:1)
The results showed that gRNAs flanked on their 5' and 3' ends with the truncated 20 nt sequence were more active in mammalian cells than those flanked by the longer 28 nt sequence (Figure 4). To the best of the present inventors' knowledge, this is the first demonstration that Csy4 nuclease can be used to process RNA transcripts in live human cells. One important additional advantage of the 20 nt truncated site is that, unlike the longer 28 nt sequence, it does not leave any additional nucleotides on the 5' end of a gRNA processed from the longer transcript (Figure 4). This enables expression of gRNAs that have ANY desired nucleotide at the 5'-most position. This is an improvement relative to expression of gRNAs from RNA polymerase III promoters which have a requirement for specific nucleotide(s) at the 5'-most position.

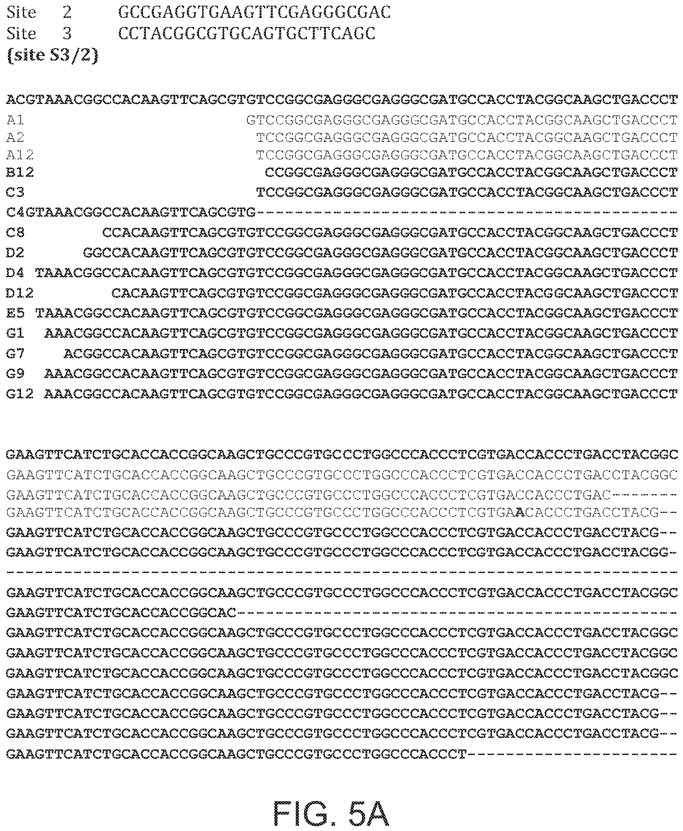

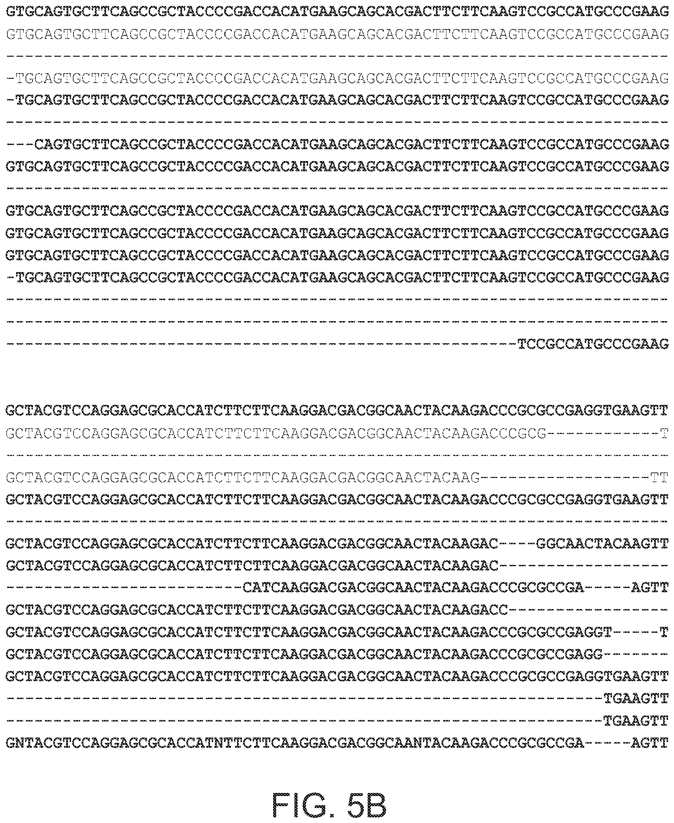

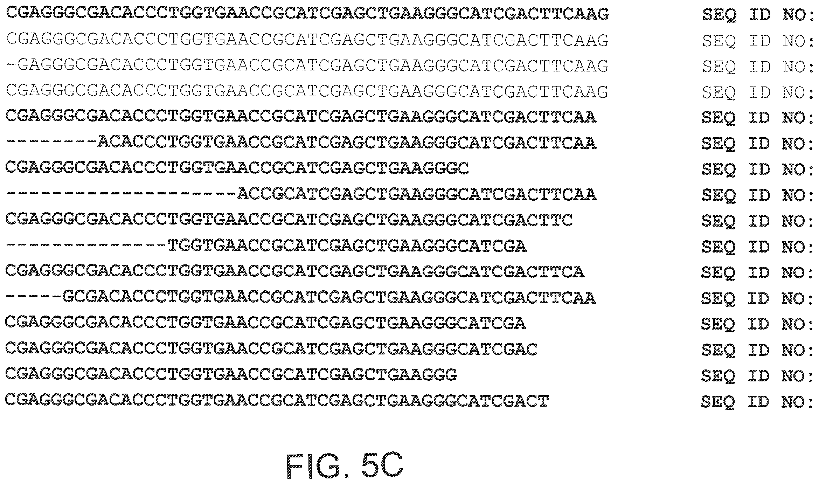

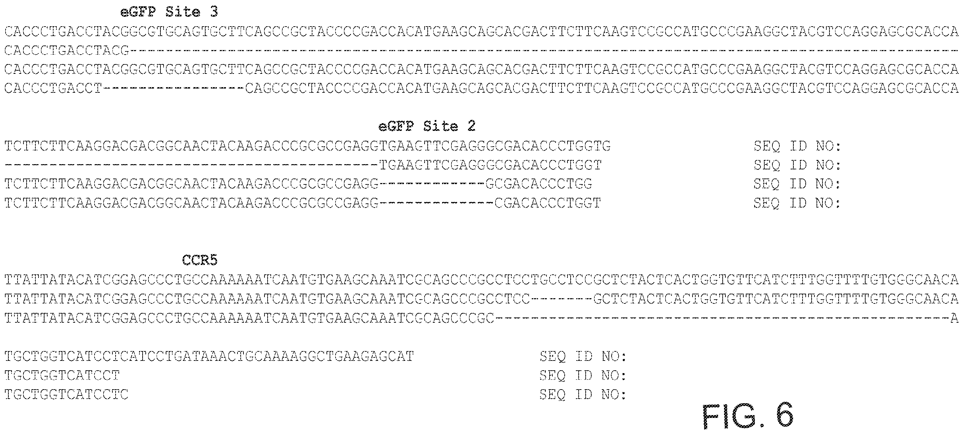

Using this Csy4-based system, the efficient expression of two and three different gRNAs (Figures 5 and 6) was demonstrated. gRNAs simultaneously expressed using this approach induced alterations at the expected sites in human cells.

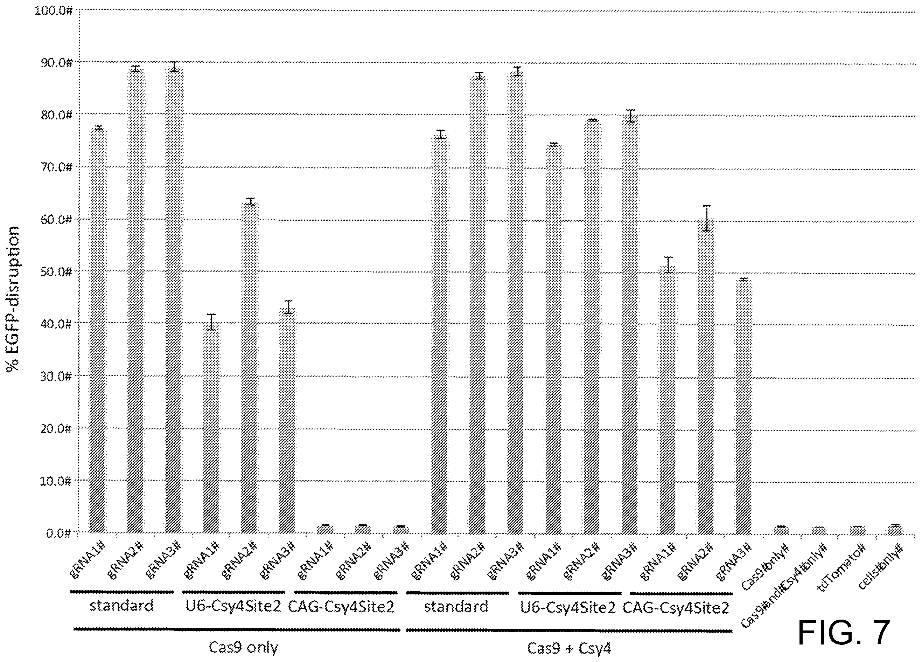

These results also demonstrated that this Csy4-based strategy could be used with gRNAs encoded on a longer mRNA produced by an RNA Pol II promoter (Figure 7). In these experiments, one of three different single gRNAs flanked by the truncated Csy4 sites was encoded on an mRNA produced by the CAG promoter (an RNA Pol II promoter). As shown in Figure 7, all three of these constructs could produce functional gRNA that could direct Cas9 nuclease in human cells but only in the presence of Csy4. The level of targeted Cas9 activity observed was comparable to (albeit somewhat lower than) what is observed when these gRNAs are expressed singly using a standard RNA Pol III promoter or as a Csy4-flanked transcript from an RNA Pol III promoter (Figure 7).

In summary, the present results demonstrated that: 1) up to three functional gRNAs can be produced from a single RNA pol III transcript when separated by Csy4 cleavage sites and in the presence of Csy4 in human cells, 2) multiple Csy4-processed gRNAs can be used to direct Cas9 nuclease to introduce multiplex changes in a single human cell, and 3) a functional gRNA flanked by Csy4 cleavage sites can be excised by Csy4 nuclease from a longer mRNA transcript made from an RNA polymerase II promoter.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A deoxyribonucleic acid (DNA) molecule comprising a plurality of sequences encoding Cas9 single guide RNAs (sgRNAs), wherein each sgRNA is flanked by at least one Csy4 cleavage sequence consisting of the sequence GTTCACTGCCGTATAGGCAG (SEQ ID NO:1).

2. The DNA molecule of claim 1, operably linked to a promoter sequence.

3. The DNA molecule of any one of claims 1 to 2, comprising two, three, or more sgRNA sequences.

4. The DNA molecule of any one of claim 1 to 3, wherein the promoter sequence is a RNA Polymerase II (Pol II) promoter or Pol III promoter.

5. The DNA molecule of claim 4, wherein the promoter sequence is a RNA Pol II promoter, and the Pol II promoter is selected from the group consisting of CAG, EF1A, CAGGS, PGK, UbiC, CMV, B29, Desmin, Endoglin, FLT-1, GFPA, and SYN1 promoters.

6. The DNA molecule of claim 1, wherein the plurality of sequences are multimeric cassettes comprising two or more sgRNA sequences, wherein each sgRNA is flanked by a Csy4 cleavage sequence, and further comprising a promoter sequence linked with the multimeric cassettes.

7. The DNA molecule of claim 6, comprising:
a Pol II promoter, operably linked to a first sequence encoding a first guide RNA linked to a Csy4 cleavage site, linked to a second sequence encoding a second guide RNA linked to a Csy4 cleavage site, linked to a third sequence encoding a third guide RNA linked to a Csy4 cleavage site.

8. The DNA molecule of claim 7, comprising a guide RNA sequence of about 100 nts.

9. The DNA molecule of any one of claims 1 to 8, wherein the sgRNA comprises the sequence:
(X₁₇₋₂₀)GUUUUAGAGCUAGAAAUAGCAAGUUAAAAUAAGGCUAGUCCG (SEQ ID NO:8); 
(X17-20)GUUUUAGAGCUAUGCUGAAAAGCAUAGCAAGUUAAAAUAAGGCUAG
UCCGUUAUC (SEQ ID NO:9);
 
(X17-20)GUUUUAGAGCUAUGCUGUUUUGGAAACAAAACAGCAUAGCAAGUUA
AAAUAAGGCUAGUCCGUUAUC (SEQ ID NO:10); or
 
(X17-20)GUUUUAGAGCUAGAAAUAGCAAGUUAAAAUAAGGCUAGUCCGUUAU
CAACUUGAAAAAGUGGCACCGAGUCGGUGC (SEQ ID NO:11),
 wherein X₁₇₋₂₀ is a sequence complementary to the complementary strand of 17-20 consecutive nucleotides of a target sequence.

10. A vector comprising the DNA molecule of claims 1-9.

11. The DNA molecule of any one of claims 1 to 9 or the vector of claim 11, further comprising a sequence encoding a functional Csy4 enzyme.

12. A method of producing a plurality of Cas9 single guide RNAs in a cell, the method comprising expressing the DNA molecule of claims 1 to 10 or 11 in the cell, wherein the cell is a mammalian cell and the cell also expresses an exogenous functional Csy4 enzyme sequence.

13. An in vitro or *ex vivo* method of altering expression of a target gene or a plurality of target genes in a cell, the method comprising expressing the DNA molecule of claims 1-11 wherein each sgRNA comprises a sequence that is complementary to at least 17-20 nts of a target gene.

## Patentansprüche

1. Desoxyribonukleinsäure-Molekül (DNA-Molekül), umfassend mehrere Sequenzen, die Cas9-Single-Guide-RNA (sgRNA) codieren, wobei jede sgRNA von mindestens einer Csy4-Spaltsequenz flankiert wird, die aus der Sequenz GTTCACTGCCGTATAGGCAG (SEQ ID Nr. 1) besteht.

2. DNA-Molekül nach Anspruch 1, das operativ mit einer Promotorsequenz verknüpft ist.

3. DNA-Molekül nach einem der Ansprüche 1 bis 2, umfassend zwei, drei oder mehr sgRNA-Sequenzen.

4. DNA-Molekül nach einem der Ansprüche 1 bis 3, wobei die Promotorsequenz ein RNA-Polymerase-II-Promotor (Pol-II-Promotor) oder ein Pol-III-Promotor ist.

5. DNA-Molekül nach Anspruch 4, wobei die Promotorsequenz ein RNA-Pol-II-Promotor ist und der Pol-II-Promotor aus der Gruppe bestehend aus CAG-, EF1A-, CAGGS-, PGK-, UbiC-, CMV-, B29-, Desmin-, Endoglin-, FLT-1-, GFPA- und SYN1-Promotoren ausgewählt ist.

6. DNA-Molekül nach Anspruch 1, wobei die mehreren Sequenzen multimere Kassetten sind, die zwei oder mehr sgRNA-Sequenzen umfassen, wobei jede sgRNA von einer Csy4-Spaltsequenz flankiert wird, und weiterhin umfassend eine Promotorsequenz, die mit den multimeren Kassetten verknüpft ist.

7. DNA-Molekül nach Anspruch 6, umfassend:
einen Pol-II-Promotor, der operativ mit einer ersten Sequenz verknüpft ist, die eine erste Guide-RNA codiert, die mit einem Csy4-Spaltort verknüpft ist, mit einer zweiten Sequenz verknüpft ist, die eine zweite Guide-RNA codiert, die mit einem Csy4-Spaltort verknüpft ist, mit einer dritten Sequenz verknüpft ist, die eine dritte Guide-RNA codiert, die mit einem Csy4-Spaltort verknüpft ist.

8. DNA-Molekül nach Anspruch 7, umfassend eine Guide-RNA-Sequenz von etwa 100 NTS.

9. DNA-Molekül nach einem der Ansprüche 1 bis 8, wobei die sgRNA die Sequenz umfasst:
(X₁₇₋₂₀)GUUUUAGAGCUAGAAAUAGCAAGUUAAAAUAAGGCUAGUCCG (SEQ ID Nr. 8); 
(X17-
20)GUUUUAGAGCUAUGCUGAAAAGCAUAGCAAGUUAAAAUAAGGCUAGUCCGUUAUC
(SEQ ID Nr. 9);
 
(X17-
20)GUUUUAGAGCUAUGCUGUUUUGGAAACAAAACAGCAUAGCAAGUUAAAAUAAGGC
UAGUCCGUUAUC (SEQ ID Nr. 10) oder
 
(X17-
20)GUUUUAGAGCUAGAAAUAGCAAGUUAAAAUAAGGCUAGUCCGUUAUCAACUUGAA
AAAGUGGCACCGAGUCGGUGC (SEQ ID Nr. 11),
 wobei X₁₇₋₂₀ eine Sequenz ist, die komplementär zu dem komplementären Strang von 17-20 aufeinanderfolgenden Nukleotiden einer Zielsequenz ist.

10. Vektor, umfassend das DNA-Molekül nach den Ansprüchen 1-9.

11. DNA-Molekül nach einem der Ansprüche 1 bis 9 oder Vektor nach Anspruch 11, weiterhin umfassend eine Sequenz, die ein funktionelles Csy4-Enzym codiert.

12. Verfahren zur Produktion von mehreren Cas9-Single-Guide-RNA in einer Zelle, wobei das Verfahren ein Exprimieren des DNA-Moleküls nach den Ansprüchen 1 bis 10 der 11 in der Zelle umfasst, wobei die Zelle eine Säugetierzelle ist und die Zelle außerdem eine exogene funktionelle Csy4-Enzym-Sequenz exprimiert.

13. In-vitro- oder Ex-vivo-Verfahren zur Modifikation einer Expression von einem Zielgen oder mehreren Zielgenen in einer Zelle, wobei das Verfahren ein Exprimieren des DNA-Moleküls nach den Ansprüchen 1-11 umfasst, wobei jede sgRNA eine Sequenz umfasst, die komplementär zu mindestens 17-20 NTS eines Zielgens ist.

## Revendications

1. Une molécule d'acide désoxyribonucléique (ADN) comprenant une pluralité de séquences codant des ARN guides uniques (sgARN) Cas9, dans laquelle chaque sgARN est flanqué d'au moins une séquence de clivage de Csy4 constituée par la séquence GTTCACTGCCGTATAGGCAG (SEQ ID NO:1).

2. La molécule d'ADN selon la revendication 1, lié de manière fonctionnelle à une séquence promotrice.

3. La molécule d'ADN selon l'une quelconque des revendications 1 à 2, comprenant deux, trois, ou plus de trois, séquences sgARN.

4. La molécule d'ADN selon l'une quelconque des revendications 1 à 3, dans laquelle la séquence promotrice est un promoteur polymérase II (Pol II) ou un promoteur Pol III de l'ARN.

5. La molécule d'ADN selon la revendication 4, dans laquelle la séquence promotrice est un promoteur Pol II, et le promoteur Pol II est sélectionné dans le groupe constitué par les promoteurs CAG, EF1A, CAGGS, PGK, UbiC, CMV, B29, Desmin, Endoglin, FLT-1, GFPA et SYN1.

6. La molécule d'ADN selon la revendication 1, dans laquelle la pluralité de séquences sont des cassettes multimères comprenant deux ou plus de deux séquences sgARN, dans laquelle chaque sgARN est flanqué par une séquence de clivage de Csy4, et comprenant en outre une séquence promotrice liée à les cassettes multimères.

7. La molécule d'ADN selon la revendication 6, comprenant :
un promoteur Pol II, lié de manière fonctionnelle à une première séquence codant un premier ARN guide lié à un site de clivage de Csy4, lié à une deuxième séquence codant un deuxième ARN guide lié à un site de clivage de Csy4, lié à une troisième séquence codant un troisième ARN guide lié à un site de clivage de Csy4.

8. La molécule d'ADN selon la revendication 7, comprenant une séquence d'ARN guide d'environ 100 nts.

9. La molécule d'ADN selon l'une quelconque des revendications 1 à 8, dans laquelle le sgARN comprend la séquence :
(X₁₇₋₂₀)GUUUUAGAGCUAGAAAUAGCAAGUUAAAAUAAGGCUAGUCCG (SEQIDNO:8) ; 
(X17-20)GUUUUAGAGCUAUGCUGAAAAGCAUAGCAAGUUAAAAUAAGGCUAG
UCCGUUAUC (SEQ ID NO:9) ;
 
(X17-20)GUUUUAGAGCUAUGCUGUUUUGGAAACAAAACAGCAUAGCAAGUUA
AAAUAAGGCUAGUCCGUUAUC (SEQ ID NO: 10) ; ou
 
(X17-20)GUUUUAGAGCUAGAAAUAGCAAGUUAAAAUAAGGCUAGUCCGUUAU
CAACUUGAAAAAGUGGCACCGAGUCGGUGC (SEQ ID NO: 11),
 dans laquelle X₁₇₋₂₀ est une séquence complémentaire au brin complémentaire de 17-20 nucléotides consécutives d'une séquence cible.

10. Un vecteur comprenant la molécule d'ADN selon l'une des revendications 1-9.

11. La molécule d'ADN selon l'une quelconque des revendications 1 à 9 ou le vecteur selon la revendication 11, comprenant en outre une séquence codant une enzyme Csy4 fonctionnelle.

12. Un procédé de production d'une pluralité d'ARN guides uniques Cas9 dans une cellule, le procédé consistant à exprimer la molécule d'ADN selon l'une des revendications 1 à 10 ou 11 dans la cellule, dans lequel la cellule est une cellule de mammifère et la cellule exprime aussi une séquence d'enzymes Csy4 fonctionnelles exogènes.

13. Un procédé in vitro ou *ex vivo* de modification d'expression d'un gène cible ou d'une pluralité de gènes cibles dans une cellule, le procédé consistant à exprimer la molécule d'ADN selon l'une des revendications 1-11, dans lequel chaque sgARN comprend une séquence qui est complémentaire d'au moins 17-20 nts d'un gène cible.
